(19) Europäisches Patentamt
European Patent Office
Office européen des brevets

(11) Publication number: 0 101 052
B1

(12) EUROPEAN PATENT SPECIFICATION

(45) Date of publication of patent specification: 20.04.88

(51) Int. Cl.$^4$: C 12 P 17/14, C 07 D 277/06

(21) Application number: 83107857.1

(22) Date of filing: 09.08.83

# (54) Method for producing 2-substituted-thiazolidine-4-carboxylic acids.

(30) Priority: 09.08.82 JP 138334/82
09.08.82 JP 138335/82
09.08.82 JP 138336/82
10.08.82 JP 138977/82
29.11.82 JP 209156/82
28.12.82 JP 228304/82

(43) Date of publication of application:
22.02.84 Bulletin 84/08

(45) Publication of the grant of the patent:
20.04.88 Bulletin 88/16

(84) Designated Contracting States:
CH DE FR GB IT LI

(56) References cited:
DE-A-1 965 343
FR-A-2 290 423
US-A-3 796 721

(73) Proprietor: AJINOMOTO CO., INC.
5-8, Kyobashi 1-chome, Chuo-ku
Tokyo 104 (JP)

(72) Inventor: Yokozeki, Kenzo
No. 1523-4 Shinsaku Takatsu-ku
Kawasaki-shi Kanagawa-ken (JP)
Inventor: Kubota, Koji
No. 6-4-6, Nagao Tama-ku
Kawasaki-shi Kanagawa-ken (JP)
Inventor: Kamimura, Akira
No. 2-25-19, Ikego
Zushi-shi Kanagawa-ken (JP)
Inventor: Eguchi, Chikahiko
No. 2136-10, Kamigo-cho Totsuka-ku
Yokohama-shi Kanagawa-ken (JP)

(74) Representative: Schübel-Hopf, Ursula et al
Strehl, Schübel-Hopf, Schulz Patentanwälte
Widenmayerstrasse 17
D-8000 München 22 (DE)

EP 0 101 052 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a method for producing a 2-substituted thiadiazolidine-4-carboxylic acid (abbreviated as STC hereinafter), which is a condensation product of a carbonyl compound and cysteine.

It has already been known from FR—2 290 423 that L-cysteine may be produced by enzymatically cleaving 2-amino-thiazoline-4-carboxylic acid in the presence of the enzyme of a micro-organism of the genus Sarcina, Achromobacter, Alcaligenes, Bacillus etc.

On the other hand it has been known from the literature, especially from J. P. Greenstein and M. Winitz, Chemistry of the Amino Acids, John Wiley and Sons, 1961, p. 1906 to 1908, that cysteine may be reacted with certain aldehydes to form the corresponding 2-substituted thiazolidine-4-carboxylic acids.

In order to obtain a 2-substituted thiazolidine-4-carboxylic acid, starting from 2-amino-thiazoline-4-carboxylic acid two separate process steps have been known, each requiring specific reaction conditions and a reaction medium, which is adjusted to the optimum conditions, especially in case of the enzymatic cleavage in which the reaction medium must be suitable for the effectiveness of the enzyme. Another difficulty is that a process using these two reactions results in a rather low yield of the final product.

It is the problem underlying the present invention to develop a method for effectively producing STC in high yields and without using tedious process steps.

Surprisingly it has been found that the above stated two reactions may be carried out simultaneously in the same reaction medium, which could not be expected, since it is a well known fact even in the general field of organic chemistry, that various difficulties arise when a two-step process, whose both reactions may easily be carried out separately, is to be conducted as a so-called one-pot reaction, since normally the reaction conditions interfere and side reactions tend to occur.

Such difficulties are especially to be expected in case one of the reactions depends on the action of a microbial enzyme. Usually the action of an enzyme strictly depends on the specific conditions of the reaction medium and may be inhibited by the presence of various chemical compounds.

By the present invention there is provided a method for producing a 2-substituted thiazolidine-4-carboxylic acid, which comprises holding 2-amino-thiazoline-4-carboxylic acid and a carbonyl compound of the following formula (I) in aqueous solution in the presence of an effective amount of an enzyme of a microorganism, said enzyme being capable of converting 2-amino-thiazoline-4-carboxylic acid to L-cysteine and said microorganism being capable of growing in a medium containing 2-amino-thiazoline-4-carboxylic acid as a nitrogen source and producing said enzyme

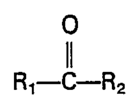

(I)

wherein $R_1$ and $R_2$ are identical or different and each represents a hydrogen atom, a formyl group, a carboxyl group, a substituted or unsubstituted saturated or unsaturated alkyl group, or a substituted or unsubstituted aryl group, with the exception of formaldehyde or other carbonyl compounds which inhibit the said enzyme.

Surprisingly, according to the present invention it has been found that not only the two reactions proceed smoothly simultaneously in the same reaction medium, but that moreover the yields obtained exceed by far the yields obtainable when the two reactions are carried out separately.

When $R_1$ or $R_2$ represents a formyl group, an example of the carbonyl compound of formula (I) is glyoxal. Examples of the carbonyl compound of formula (I) wherein $R_1$, $R_2$ or both represent a carboxyl group include pyruvic acid, α-ketoglutaric acid, glucuronic acid, glyoxylic acid and ascorbic acid. Examples of the carbonyl compound of formula (I) in which $R_1$, $R_2$ or both represent a substituted or unsubstituted saturated or unsaturated alkyl group include acetone, methyl ethyl ketone, diisobutyl ketone, fructose, glucose, arabinose, xylose, rhamnose, gluconolactone, pyruvic acid, ascorbic acid, acetaldehyde, glucuronic acid, benzaldehyde, and α-phenylpropionaldehyde. Examples of the carbonyl compound of formula (I) in which $R_1$, $R_2$ or both represent a substituted or unsubstituted aryl group include phenyl methyl ketone, diphenyl ketone, salicyclaldehyde, and hydroxynaphthaldehyde.

Formaldehyde as the above carbonyl compound, however, inhibits an enzyme of a microorganism, which has the ability to convert ATC to L-cysteine. Hence, even when ATC and formaldehyde are held in aqueous solution in the presence of the enzyme, the corresponding STC, i.e. thiazolidine-4-carboxylic acid (thioproline), is not formed. When it is desired to produce STC corresponding to a compound, such as formaldehyde, which inhibits an enzyme of a microorganism having the ability to convert ATC to L-cysteine, this inhibiting compound is reacted in aqueous solution with 2,2-dimethyl-thiazolidine-4-carboxylic acid (DTC for short), 2-methyl-thiazolidine-4-carboxylic acid (MTC for short) or 2-methyl-2-ethyl-thiazolidine-4-carboxylic acid (METC for short) to obtain the desired STC. DTC, MTC or METC can be efficiently produced by the method of this invention. Specifically, such a compound can be produced by holding ATC and acetone, acetaldehyde or methyl ethyl ketone in aqueous solution in the presence of an enzyme of a microorganism, which has the ability to convert ATC to L-cysteine.

According to another embodiment of the present invention there is provided a process for producing a 2-substituted thiazolidine-4-thiazoline-4-carboxylic acid which comprises the step of holding 2-amino-

thiazoline-4-carboxylic acid and a first carbonyl compound, selected from acetone, acetaldehyde or methylethylketone in the presence of an effective amount of an enzyme of a microorganism, said enzyme being capable of growing in a medium containing 2-amino-thiazoline-4-carboxylic acid as a nitrogen source and producing said enzyme and which method further comprises an additional step of holding the 2-substituted-thiazoline-4-carboxylic acid prepared by said reaction in the presence of an enzyme inhibiting amount of a second carbonyl compound, wherein a second 2-substituted thiazolidine-4-carboxylic acid is produced.

Hydrolysis of STC under acidic conditions can produce L-cysteine quantitatively within a short period of time. This method is a superior method for producing L-cysteine to the method described in U.S. Patent No. 4,006,057. Reaction of STC with a halogenoacetic acid under alkaline conditions can give S-carboxymethyl-L-cysteine (to be abbreviated as SMC hereinafter). SMC can be produced efficiently within a short period of time if STC is DTC, MEC or METC.

L-cysteine has various antidotal activities, and is used as an antidote. It is also used as an agent for treating bronchitis, a cosmetica and a hair nourishing agent. SMC is used as an agent for treating bronchitis, an expectorant, and an antidote. Many STC compounds obtained by the condensation of L-cysteine and carbonyl compounds are useful as medicines. For example, a cysteine-glucuronic acid condensate is used as an anticataract agent (see Japanese Laid-Open Patent Publication No. 18615/1982). It is known that condensates of sugars and cysteine, particularly a condensate of glucose and L-cysteine, can be used as an agent for preventing and treating radioactive ray troubles or as food fortifiers (Japanese Patent Publication No. 23457/1968). It is also known that 2-phenylthiazolidine-4-carboxylic acid which is a condensation product of benzaldehyde and L-cysteine can be used as an antitumor agent against solid tumors (Japanese Laid-Open Patent Publication No. 167220/1980), and condensates of aromatic or aliphatic aldehydes and L-cysteins, for example 2-(4-methoxyphenyl)-thiazolidine-4-carboxylic acid and 2-heptyl-thiazolidine-4-carboxylic acid, can be used as anticancer agents (Japanese Laid-Open Patent Publication No. 128625/1982). It is further known that a glyoxal/cysteine condensate can be used as an antitumor agent (Japanese Laid-Open Patent Publication No. 167960/1982).

The microorganisms used in this invention have a high ability to produce an enzyme capable of converting ATC to L-cysteine. Examples of the microorganism are those which have a high ability to produce an enzyme capable of converting ATC to L-cysteine and belong to genera *Achromobacter, Alcaligenes, Bacillus, Brevibacterium, Enterobacter, Erwinia, Escherichia, Flabobacterium, Micrococcus, Mycoplana, Pseudomonas, Sarcina* and *Serratia* described, for example, in U.S. Patent No. 4,006,057.

Specific examples of the microorganisms are the following strains.

| | |
|---|---|
| *Sarcina lutea* | ATCC 272 |
| *Achromobacter delmarvae* | FERM—P 3483 |
| *Alcaligenes denitrificans* | ATCC 15173 |
| *Bacillus brevis* | ATCC 8185 |
| *Brevibacterium flavum* | ATCC 13826 |
| *Enterobacter aerogenes* | FERM—P 321 |
| *Erwinia carotovora* | FERM—P 2766 |
| *Pseudomonoas thiazolinophilum* | FERM—BP 322 |
| *Pseudomonas desmolytica* | FERM—BP 323 |
| *Escherichia coli* | FERM—P 2763 |
| *Micrococcus sodonensis* | ATCC 11880 |
| *Mycoplana dimorpha* | ATCC 4249 |
| *Serratia marcescens* | FERM—P 2765 |
| *Flavobacterium acidoficum* | ATCC 8366 |
| *Pseudomonas ovalis* | FERM—P 2762 |

To cultivate the microorganism, there is used a culture medium which contains a carbon source, a nitrogen source and inorganic ions and if required, very small amounts of organic nutrients. Examples of

the carbon source are sugars such as glucose, sucrose, xylose and molasses, organic acids such as acetic acid, and alcohols such as ethanol, glycerol and methanol. Examples of the nitrogen source are ammonium sulfate and ammonium chloride. Examples of the organic nutrients are yeast extract, peptone, meat extract and corn steep liquor. Examples of the inorganic ions are ions of magnesium, iron, manganese, potassium, sodium and phosphoric acid.

The microorganism is cultivated in accordance with a conventional method. For example, it is cultivated aerobically in the culture medium kept at a pH of 6 to 9 and a temperature of 20 to 40°C.

Desirably, a small amount of ATC is added at the time of cultivation. In this way, a microorganism strain having a high ability to produce an enzyme capable of converting ATC to L-cysteine can be obtained. The enzyme that can be used in this invention may, for example, be the culture broth obtained by such cultivation, microbial cells separated from the culture broth, living cells obtained by washing, lyophilized cells, cells dried over acetone, chemically or biochemically broken cells, extracts, crude products, purified products, purified protein products, and fixed products of cells or purified cells.

The starting ATC may be any of D-isomer, L-isomer, and a racemic mixture supplied by synthetic methods.

The concentrations of the starting ATC and carbonyl compound in the aqueous solution vary according to whether the method is operated batchwise or continuously. In the case of the batchwise method, their concentrations are generally about 0.1 to about 30%, preferably about 0.5 to about 10%, in the aqueous solution. In the continuous method, their concentrations are preferably a little bit lower than those in the batchwise method.

The reaction is carried out in an aqueous solution containing 2 to 40%, preferably 5 to 20%, of the carbonyl compound. The addition of hydroxylamine or semicarbazide can increase the yield of the desired product. The reaction temperature is 15 to 60°C, preferably 30 to 50°C, and the pH is in the range of 6 to 10, preferably 7.0 to 9.5.

By performing the reaction in this way, cysteine is formed in the reaction mixture, and this cysteine is condensed with the carbonyl compound to form STC. Unlike cysteine, the resulting STC does not at all inhibit hydrolysis of ATC to cysteine. Hence, the yield of STC is high, and a period of 1 to 25 hours is a sufficient reaction time. This period is about one-half of the time required to hydrolyse ATC to cysteine which is 2 to 50 hours. Furthermore, according to the method of this invention, the generation of hydrogen sulfide by an enzyme detrimental to cysteine can be prevented. The method of this invention is therefore very advantageous industrially.

The following Examples illustrate the present invention more specifically.

Example 1

A culture medium having the composition shown in Table 1 was prepared, dividedly put into 500 ml shaking flasks in a volume of 50 ml for each, and heated at 120°C for 10 minutes.

TABLE 1

Composition of the culture medium (pH 7.0)

| Component | Content |
|---|---|
| Glycerol | 1.0% |
| Yeast extract | 0.5% |
| Peptone | 0.5% |
| Meat extract | 0.5% |
| NaCl | 0.5% |
| DL—ATC | 0.2% |

*Pseudomonas desmolytica* AJ 3868, FERM—BP 323 was inoculated in the culture medium, and cultivated with shaking at 30°C for 24 hours. The culture broth was centrifuged and to collect the microbial cells, 5.0 g of the wet cells were added to 100 ml of a substrate solution having the composition shown in Table 2, and reacted for 16 hours at 30°C with occasional light stirring.

TABLE 2

Composition of the substrate solution (pH 8.0)

| Component | Content |
|---|---|
| DL—ATC | 2.0 g/dl |
| $KH_2PO_4$ | 1.0 g/dl |
| Hydroxylamine hydrochloride | 0.15 g/dl |
| Acetone | 0—50% (V/V) |

After the reaction, the reaction mixture was subjected to silica gel thin-layer chromatography (developing solvent, tertiary butanol:methyl ethyl ketone:water:$NH_4OH$=4:3:2:1) and DTC was quantitatively determined by using a densitometer (210 nm). The results are shown in Table 3.

Table 3 also shows the amounts of L-cysteine formed by adding hydrochloric acid to each of the reaction mixtures after the reaction to adjust its pH to 3.0.

TABLE 3

Amounts of DTC and L-cysteine formed

| Concentration of acetone (%) | DTC (g/dl) | L-cysteine (g/dl) |
|---|---|---|
| 0 | 0 | 0.32 |
| 2 | 1.62 | 1.20 |
| 5 | 1.67 | 1.24 |
| 10 | 1.68 | 1.25 |
| 15 | 1.68 | 1.25 |
| 20 | 1.57 | 1.16 |
| 30 | 1.41 | 1.06 |
| 50 | 0.72 | 0.50 |

Example 2

5.0 g of the wet cells of *Pseudomonas desmolytica* FERM—BP 323 obtained by the method of Example 1 were added to 100 ml of a substrate solution having the same composition as shown in Table 2 (except that the amount of acetone was 5.0%), and reacted by maintaining the mixture at 30°C for 16 hours.

Sodium hydroxide was added to the reaction mixture to adjust its pH to 9.0, and it was concentrated to dryness under reduced pressure. The dried mixture was put into 500 ml of acetone, and after sufficient stirring, insoluble materials were separated by filtration. The solution was concentrated to 100 ml at 60°C. Hydrogen chloride gas was added to the solution to lower its pH to 3.0, and the solution was left to stand at 8°C for a day and night to precipitate crystals. The crystals were recrystallized by the same procedure to obtain 0.7 g of crystals.

The recrystallized crystals showed the following elemental analysis values. C: 44.82, H: 6.86, N: 8.77, S: 19.89(%). They had a melting point of 134 to 134.5°C, and completely correspond to an authentical sample of DTC. Thus, the resulting crystals were determined to be DTC.

Example 3

5.0 g of the wet cells obtained by the method of Example 1 were lyophilized (sample 1).

5.0 g of the same wet cells were suspended in 50 ml of a 0.1M phosphate buffer (pH 7.0) and the suspension was subjected to an ultrasonication treatment (Model LIR—200P made by Tomy Seiko Co., Ltd.; 20 KC, 5 minutes) (sample 2).

5.0 g of the same wet cells were suspended in 20 ml of deionized water, and 3.8 g of acrylamide and 225 mg of methylenebis-acrylamide were added. $N_2$ gas was passed through the mixture to remove oxygen, and 17.5 mg of ammonium persulfate and 40 μg of N',N'-dimethylaminopropionitrile were added to fix the cells (sample 3).

Each of samples 1, 2 and 3 was added to 100 ml of a substrate solution having the same composition as shown in Table 2 (except that the amount of acetone was 5.0%) and enzymatically reacted by maintaining the mixture at 30°C for 16 hours. After the reaction, insoluble materials were removed from the reaction mixture, and DTC was quantitatively determined by the same method as in Example 1. The results are shown in Table 4.

TABLE 4

Amount of DTC formed

| Treated cell product | DTC (g) |
|---|---|
| Sample 1 | 1.67 |
| Sample 2 | 1.69 |
| Sample 3 | 1.42 |

Example 4

Each of the microorganisms shown in Table 5 was cultivated in the culture medium shown in Table 1 by the method of Example 1. The culture broth was centrifuged to collect the microbial cells. 5.0 g of wet cells were taken, and added to 100 ml of a substrate solution having the same composition as shown in Table 2 (except that the amount of acetone was 5.0%). They were reacted by maintaining the mixture at 30°C for 24 hours. After the reaction, DTC in the reaction mixture was quantitatively determined by the method described in Example 1. The results are shown in Table 5.

TABLE 5

Amount of DTC formed

| Microorganism strain | DTC (g/dl) |
|---|---|
| S. lutea ATCC 272 | 0.18 |
| A. delmarvae FERM—P 3483 | 0.16 |
| A. denitrificans ATCC 15173 | 0.66 |
| B. brevis ATCC 8185 | 0.18 |
| Brevi. flavum ATCC 13826 | 0.12 |
| Ent. aerogenes FERM—EP 321 | 0.34 |
| E. carotovora FERM—P 2766 | 0.11 |
| E. coli FERM—P 2763 | 0.13 |
| M sodonensis ATCC 11880 | 0.99 |
| Myco. dimorpha ATCC 4279 | 0.32 |
| S. marcescens FERM—P 2765 | 0.16 |
| F. acidoficum ATCC 8366 | 0.13 |
| Pseu. ovalis FERM—P 2762 | 0.82 |
| Pseu. thiazolinophilum FERM—BP 322 | 0.79 |

Example 5

A culture medium having the composition shown in Table 1 was prepared, dividedly put into 500 ml shaking flasks in a volume of 50 ml for each, and heated at 120°C for 10 minutes.

*Pseudomonas desmolytica* AJ 3868 FERM—BP 323 was inoculated in the culture medium and cultivated at 30°C for 24 hours with shaking. The culture broth was centrifuged to collect the microbial cells. 5.0 g of the wet cells were added to 100 ml of a substrate solution having the composition shown in Table 6, and reacted for 16 hours by maintaining the mixture at 30°C with occasional light stirring.

TABLE 6

Composition of the substrate solution (pH 8.0)

| Component | Content |
|---|---|
| DL—ATC | 2.0 g/dl |
| $KH_2PO_4$ | 1.0 g/dl |
| Hydroxylamine hydrochloride | 0.15 g/dl |
| Methyl ethyl ketone or acetaldehyde | 5.0% (V/V) |

After the reaction, the reaction mixture was subjected to silica gel thin-layer chromatography (developing solvent, tertiary butanol:methyl ethyl ketone:water:$NH_4OH$=4:3:2:1), and DTC, METYC or MTC was quantitatively determined by using a densitometer (210 nm). It was found that 1.23 g/dl of METC or 1.41 g/dl of MTC was formed in the reaction mixture.

Example 6

A culture medium having the composition shown in Table 1 was prepared, dividely put into 500 ml shaking flasks in a volume of 50 ml for each, and heated at 120°C for 10 minutes.

*Pseudomonas desmolytica* AJ 3868 FERM—BP 323 was inoculated in the culture medium, and cultivated at 30°C for 24 hours with shaking. The culture broth was centrifuged to collect the microbial cells. 5.0 g of wet cells were added to 100 ml of a substrate solution having the composition shown in Table 2, and reacted for 16 hours by maintaining the mixture at 30°C with occasional light stirring.

After the reaction, 3.3 g of sodium monochloroacetate was added to the reaction mixture, and a solution of sodium hydroxide was added to adjust its pH to 13.5. The mixture was maintained at 30°C for 10 hours to form carboxymethylcysteine. The amount of carboxymethylcysteine was determined by high-performance liquid chromatography using a cation exchange resin "ZYPAX SCX". The results are shown in Table 7.

TABLE 7

Amount of carboxymethylcysteine formed

| Amount of acetone (%) | SMC (g/dl) |
|---|---|
| 0 | 0.9 |
| 2 | 2.0 |
| 5 | 2.2 |
| 10 | 2.3 |
| 15 | 2.3 |
| 20 | 1.8 |
| 30 | 1.4 |

The reaction mixture obtained by performing the enzymatic reaction as above at an acetone concentration of 5.0% was reacted in the same way as above except that monofluoroacetic acid, monoiodoacetic acid or monobromoacetic acid was used instead of sodium monochloroacetate. The

amount of carboxymethylcysteine formed was 2.2 g, 2.2 g, or 2.3 g, respectively. There was scarcely any difference observed.

1.3 g of DTC and 1.92 of monochloroacetic acid were added to 70 ml of water, and the pH of the solution was adjusted to 6.0—14.0 by using a sodium hydroxide solution. The resulting solutions having the various pH values were each mixed with water to adjust the entire volume to 100 ml. Each of the solutions was maintained at 30°C for 1 hour, and the relation between the pH in the reaction and the amount of SMC formed was examined. The results are shown in Table 8.

TABLE 8

pH in the reaction and the amount of SMC

| pH | (g) | Yield (mole %) |
|---|---|---|
| 6.0 | 0.20 | 13 |
| 7.0 | 0.33 | 22.5 |
| 9.8 | 0.72 | 49.0 |
| 11.3 | 1.40 | 95.0 |
| 13.4 | 1.45 | 99.0 |
| 14.0 | 1.45 | 99.0 |

Example 7

5.0 g of the wet cells obtained by the method of Example 1 were lyophilized (sample A).

5.0 g of the same wet cells were suspended in 50 ml of a 0.1M phosphate buffer (pH 7.0) and the suspension was subjected to an ultrasonication treatment (Model LIR—200P made by Tomy Seiko Co., Ltd.; 20 KC, 5 minutes). (sample B).

5.0 g of the same wet cells were suspended in 20 ml of deionized water, and 3.8 g of acrylamide and 225 mg of methylenebis-acrylamide were added. $N_2$ gas was passed through the mixture to remove oxygen, and 17.5 mg of ammonium persulfate and 40 µg of N',N'-dimethylaminopropionitrile were added to fix the cells (sample C).

Each of samples A, B and C was added to 100 ml of a substrate solution having the same concentration as shown in Table 2 (except that the amount of acetone was 5.0%), and enzymatically reacted by maintaining the mixture at 30°C for 16 hours. After the reaction, insoluble materials were removed from the reaction mixture, and 3.3 g of sodium monochloroacetate was added. The pH of the solution was adjusted to 13.5 by adding sodium hydroxide, and it was reacted by maintaining it at 30°C for 1.0 hour. The amount of carboxymethylcysteine formed in the reaction mixture was shown in Table 9.

TABLE 9

| Treated cell product | SMC (g) |
|---|---|
| Sample A | 2.1 |
| Sample B | 2.0 |
| Sample C | 1.8 |

Example 8

Each of the microorganisms shown in Table 10 was cultivated in the culture medium shown in Table 1 by the method of Example 1. The culture broth was centrifuged to separate the microbial cells. 5.0 g of the wet cells were suspended in 100 ml of a substrate solution having the same composition as shown in Table 2 (except that the amount of acetone was 5.0%), and reacted by maintaining the suspension at 30°C for 24 hours. After the reaction, 3.3 g of sodium monochloroacetate was added to adjust the pH of the reaction mixture to 13.0, and maintained at 30°C for 1 hour to form carboxymethylcysteine. The amount of carboxymethylcysteine was determined by high-performance liquid chromatography. The results are shown in Table 10.

TABLE 10

Amount of SMC formed

| Microorganism strain | SMC (g/dl) |
|---|---|
| S. lutea ATCC 272 | 0.2 |
| A. delmarvae FERM—P 3483 | 0.2 |
| A. denitrificans ATCC 15173 | 1.3 |
| B. brevis ATCC 8185 | 0.3 |
| Brevi. Flavum ATCC 13826 | 0.1 |
| Ent. aerogenes FERM—BP 321 | 1.1 |
| E. carotovora FERM—P 2766 | 0.3 |
| E. coli FERM—P 2763 | 0.1 |
| M. sodonensis ATCC 11880 | 1.2 |
| Myco. dimorpha ATCC 4279 | 1.0 |
| S. marcescens FERM—P 2765 | 0.1 |
| F. acidoficum ATCC 8366 | 0.2 |
| Pseu. ovalis FERM—P 2762 | 1.4 |
| Pseu. thiazolinophilum FERM—BP 322 | 1.6 |

Example 9

Each of the carbonyl compounds shown in Table 11 was added in an amount of 1.0 g/dl to an aqueous solution (pH 8.0) containing 1.0 g/dl each of DTC obtained in an acetone concentration of 5.0% in Example 1 or by the method shown in Example 5 and MTC or METC obtained by a similar method. The mixture was maintained at 40°C for 2 hours to perform a substitution reaction. The condensate of L-cysteine and the carbonyl compound formed in the reaction mixture was identified and quantitatively determined. The results are shown in Tables 11 and 12.

Identification of the reaction products (condensates) was carried out by subjecting the reaction mixtures to silica gel thin-layer chromatography (n-propanol:1%$NH_4OH$=2:1), and the amounts of the reaction products were determined by using a densitometer (210 nm).

TABLE 11

Results of identification of the condensates

| Carbonyl compound | Condensate | (No.) |
|---|---|---|
| Formaldehyde | Thiazolidine-4-carboxylic acid | (1) |
| Glyoxylic acid | Cysteine-glyoxalic acid | (2) |
| Glyoxal | Cysteine-glyoxal | (3) |
| Benzaldehyde | 2-Phenyl-thiazolidine-4-carboxylic acid | (4) |
| α-Phenylpropionaldehyde | 2-(1′-Phenylethyl)-thiazolidinone-4-carboxylic acid | (5) |
| Salicylaldehyde | 2-(2′-Hydroxyphenyl)-thiazolidine-4-carboxylic acid | (6) |
| Hydroxynaphthaldehyde | 2-(2′-hydroxy-1-naphthyl)-thiazolidine-4-carboxylic acid | (7) |
| Glucose | Cysteine-glucose | (8) |
| Fructose | Cysteine-fructose | (9) |
| Xylose | Cysteine-xylose | (10) |
| Arabinose | Cysteine-arabinose | (11) |
| Rhamnose | Cysteine-rhamnose | (12) |
| α-Ketoglutaric acid | Cysteine-α-ketoglutaric acid | (13) |
| Pyruvic acid | Cysteine-pyruvic acid | (14) |
| Glucuronic acid | Cysteine-glucuronic acid | (15) |
| Ascorbic acid | Cysteine-ascorbic acid | (16) |
| Diisobutyl ketone | 2,2-Diisobutyl-thiazolidine-4-carboxylic acid | (17) |
| Gluconolactone | Cysteine-gluconolactone | (18) |
| Phenyl methyl ketone | 2-Phenyl-2-methyl-thiazolidine-4-carboxylic acid | (19) |

TABLE 12

Amount of the condensate formed (g/dl)

| Condensate formed | Starting condensate | | | |
|---|---|---|---|---|
| | ATC | DTC | METC | MTC |
| (1) | — | 0.96 | 0.78 | 0.72 |
| (2) | 0.32 | 0.32 | 0.28 | 0.26 |
| (3) | 0.28 | 0.31 | 0.26 | 0.24 |
| (4) | 0.86 | 0.41 | 0.31 | 0.29 |
| (5) | 0.52 | 0.59 | 0.47 | 0.43 |
| (6) | 0.47 | 0.54 | 0.43 | 0.40 |
| (7) | 0.43 | 0.49 | 0.40 | 0.37 |
| (8) | 0.14 | 0.13 | 0.10 | 0.10 |
| (9) | 0.16 | 0.12 | 0.11 | 0.10 |
| (10) | 0.15 | 0.12 | 0.11 | 0.10 |
| (11) | 0.13 | 0.13 | 0.12 | 0.10 |
| (12) | 0.26 | 0.16 | 0.13 | 0.12 |
| (13) | 1.21 | 0.62 | 0.52 | 0.48 |
| (14) | 0.91 | 0.58 | 0.41 | 0.37 |
| (15) | 1.24 | 0.71 | 0.66 | 0.61 |
| (16) | 0.48 | 0.31 | 0.23 | 0.21 |
| (17) | 0.12 | 0.09 | 0.08 | 0.08 |
| (18) | 1.0 | 0.65 | 0.62 | 0.64 |
| (19) | 0.48 | 0.52 | 0.46 | 0.44 |

**Claims**

1. A method for producing a 2-substituted thiazolidine-4-carboxylic acid, which comprises holding 2-amino-thiazoline-4-carboxylic acid and a carbonyl compound of the following formula (I) in aqueous solution in the presence of an effective amount of an enzyme of a microorganism, said enzyme being capable of converting 2-amino-thiazoline-4-carboxylic acid to L-cysteine and said microorganism being capable of growing in a medium containing 2-amino-thiazoline-4-carboxylic acid as a nitrogen source and producing said enzyme

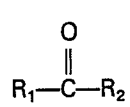

(I)

wherein $R_1$ and $R_2$ are identical or different and each represents a hydrogen atom, a formyl group, a carboxyl group, a substituted or unsubstituted saturated or unsaturated alkyl group, or a substituted or unsubstituted aryl group, with the exception of formaldehyde or other carbonyl compounds which inhibit the said enzyme.

2. A method for producing a 2-substituted thiazolidine-4-carboxylic acid, which comprises the step of holding 2-amino-thiazoline-4-carboxylic acid and a first carbonyl compound, selected from acetone, acetaldehyde or methylethylketone in the presence of an effective amount of an enzyme of a microörganism, said enzyme being capable of converting 2-amino-thiazoline-4-carboxylic acid to L-cysteine and said microorganism being capable of growing in a medium containing 2-amino-thiazoline-4-carboxylic acid as a nitrogen source and producing said enzyme and which method further comprises an additional step of holding the 2-substituted thiazolidine-4-carboxylic acid prepared by said reaction in the presence of an enzyme inhibiting amount of a second carbonyl compound, wherein a second 2-substituted thiazolidine-4-carboxylic acid is produced.

3. The method of claim 2, wherein said second carbonyl compound is formaldehyde.

4. The method of any of the Claims 1 to 3, wherein said microorganism belongs to the genus *Sarcina, Achromobacter, Alcaligenes, Bacillus, Brevibacterium, Enterobacter, Erwinia, Escherichia, Micrococcus, Mycoplana, Serratia, Flavobacterium* or *Pseudomonas.*

5. The method of Claim 4, wherein said microorganism belongs to the species *Sarcina lutea, Achromobacter delmarvae, Alcaligenes denitrificans, Bacillus brevis, Brevibacterium flavum, Enterobacter aerogenes, Erwinia carotovora, Escherichia coli, Micrococcus sodonensis, Mycoplana dimorpha, Serratia marcescens, Pseudomonas thiazolinophilum, Pseudomonas ovalis, Flavobacterium acidoficum, Pseudomonas desmolytica,* or *Pseudomonas cohaerens.*

6. The method of any of the Claims 1 to 5, wherein said microorganism is *Sarcina lutea* ATCC 272, *Alcaligenes denitrificans* ATCC 15173, *Bacillus brevis* ATCC 8185, *Brevibacterium flavum* ATCC 13826, *Enterobacter aerogenes* FERM—BP 321, *Mycoplana dimorpha* ATCC 4279, *Pseudomonas thiazolinophilum* FERM—BP 322 or *Pseudomonas desmolytica* FERM—BP 323.

**Patentansprüche**

1. Verfahren zur Herstellung einer 2-substituierten Thiazolidin-4-carbonsäure, bei dem 2-Amino-thiazolin-4-carbonsäure und eine Carbonylverbindung der nachstehenden Formel (I) in wäßriger Lösung in Gegenwert einer wirksamen Menge eines Enzyms eines Mikroorganismus gehalten werden, welches befähigt ist, 2-Amino-thiazolin-4-carbonsäure in L-Cystein umzuwandeln, wobei der Mikroorganismus befähigt ist, in einer Medium zu wachsen, welches 2-Amino-thiazolin-4-carbonsäure als Stickstoffquelle enthält und zur Bildung des Enzyms befähigt ist

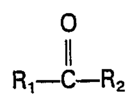 (I)

worin $R_1$ und $R_2$ gleich oder verschieden sind und jeweils für ein Wasserstoffatom, eine Formylgruppe, eine Carboxylgruppe, eine substituierte oder unsubstituierte gesättigte oder ungesättigte Alkylgruppe oder eine substituierte oder unsubstituierte Arylgruppe stehen, ausgenommen Formaldehyd oder andere Carbonylverbindungen, welche das angegebene Enzym inhibieren.

2. Verfahren zur Herstellung einer 2-substituierten Thiazolidin-2-carbonsäure, welches eine Stufe, in der 2-Amino-thiazolen-4-carbonsäure und eine erst Carbonylverbindung, gewählt unter Aceton, Acetaldehyd oder Methylethylketon, in Gegenwert einer wirksamen Menge eines Enzyms eines Mikroorganismus gehalten werden, wobei das Enzym befähigt ist, 2-Amino-thiazolin-4-carbonsäure in L-Cystein umzuwandeln und der Mikroorganismus befähigt ist, in einem Medium zu wachsen, das 2-Amino-thiazolin-4-carbonsäure als Stickstoffquelle enthält und befähigt ist, das Enzym zu bilden, und eine zusätzliche Stufe umfaßt, in der die durch diese Reaktion hergestellte 2-substituierte Thiazolidin-4-carbonsäure in Gegenwert einer Enzym-inhibierenden Menge einer zweiten Carbonylverbindung gehalten wird, wobei eine zweite 2-substituierte Thiazolidin-4-carbonsäure hergestellt wird.

3. Verfahren nach Anspruch 2, bei dem die zweite Carbonylverbindung Formaldehyd ist.

4. Verfahren nach einem der Ansprüche 1 bis 3, bei dem der Mikroorganismus dem Genus *Sarcina, Achromobacter, Alcaligenes, Bacillus, Brevibacterium, Enterobacter, Erwinia, Escherichia, Micrococcus, Mycoplana, Serratia, Flavobacterium* oder *Pseudomonas* angehört.

5. Verfahren nach Anspruch 4, bei dem der Mikroorganismus zur Species *Sarcina lutea, Achromobacter delmarvae, Alcaligenes denitrificans, Bacillus brevis, Brevibacterium flavum, Enterobacter aerogenes, Erwinia carotovora, Escherichia coli, Micrococcus sodonensis, Mycoplana dimorpha, Serratia marcescens, Pseudomonas thiazolinophilum, Pseudomonas ovalis, Flavobacterium acidoficum, Pseudomonas desmolytica,* oder *Pseudomonas cohaerens* gehört.

6. Verfahren nach einem der Ansprüche 1 bis 5, bei dem der Mikroorganismus *Sarcina lutea* ATCC 272, *Alcaligenes denitrificans* ATCC 15173, *Bacillus brevis* ATCC 8185, *Brevibacterium flavum* ATCC 13826, *Enterobacter aerogenes* FERM—BP 321, *Mycoplana dimorpha* ATCC 4279, *Pseudomonas thiazolinophilum* FERM—BP 322 oder *Pseudomonas desmolytica* FERM—BP 323 ist.

**Revendications**

1. Un procédé de production d'un acide thiazolidine-4-carboxylique 2-substitué qui comprend le maintien d'acide 2-amino-thiazoline-4-carboxylique et d'un composé carbonylé répondant à la formule (I) suivante en solution aqueuse en présence d'une quantité efficace d'une enzyme d'un micro-organisme, ladite enzyme étant capable de transformer l'acide 2-amino-thiazoline-4-carboxylique en L-cystéine et ledit micro-organisme étant capable de se développer dans un milieu contenant de l'acide 2-amino-thiazoline-4-carboxylique comme source d'azote et de produire ladite enzyme

$$R_1-\overset{\overset{\displaystyle O}{\|}}{C}-R_2 \qquad (I)$$

dans laquelle $R_1$ et $R_2$ sont identiques ou différents et représentent chacun un atome d'hydrogène, un groupe formyle, un groupe carboxyle, un groupe alkyle saturé ou insaturé substitué ou non substitué ou un groupe aryle substitué ou non substitué, à l'exception du formaldéhyde ou des autres composés carbonylés qui inhibent ladite enzyme.

2. Un procédé pour la production d'un acide thiazolidine-4-carboxylique 2-substitué qui comprend le stade de maintien de l'acide 2-amino-thiazoline-4-carboxylique et d'un premier composé carbonylé choisi parmi l'acétone, l'acétaldéhyde ou la méthyléthylcétone en présence d'une quantité efficace d'une enzyme d'un micro-organisme, ladite enzyme étant capable de transformer l'acide 2-amino-thiazoline-4-carboxylique en L-cystéine et ledit micro-organisme étant capable de se développer dans un milieu contenant de l'acide 2-amino-thiazoline-4-carboxylique comme source d'azote et de produire ladite enzyme, lequel procédé comprend de plus un stade additionnel de maintien de l'acide thiazolidine-4-carboxylique 2-substitué préparé par ladite réaction en présence d'une quantité inhibant l'enzyme d'un second composé carbonylé pour produire un second acide thiazolidine-4-carboxylique 2-substitué.

3. Le procédé de la revendication 2, dans lequel ledit second composé carbonylé et le formaldéhyde.

4. Le procédé de l'une quelconque des revendications 1 à 3, dans lequel ledit micro-organisme appartient aux genres *Sarcina, Achromobacter, Alcaligenes, Bacillus, Brevibacterium, Enterobacter, Erwinia, Escherichia, Micrococcus, Mycoplana, Serratia, Flavobacterium* ou *Pseudomonas.*

5. Le procédé de la revendication 4, dans lequel ledit micro-organisme appartient aux espèces *Sarcina lutea, Achromobacter delmarvae, Alcaligenes denitrificans, Bacillus brevis, Brevibacterium flavum, Enterobacter aerogenes, Erwinia carotovora, Escherichia coli, Micrococcus sodonensis, Mycoplana dimorpha, Serratia marcescens, Pseudomonas thiazolinophilum, Pseudomonas ovalis, Flavobacterium acidoficum, Pseudomonas desmolytica* ou *Pseudomonas cohaerens.*

6. Le procédé de l'une quelconque des revendications 1 à 5 dans lequel ledit micro-organisme est *Sarcina lutea* ATCC 272, *Alcaligenes denitrificans* ATCC 15173, *Bacillus brevis* ATCC 8185, *Brevibacterium flavum* ATCC 13826, *Enterobacter aerogenes* FERM—BP 321, *Mycoplana dimorpha* ATCC 4279, *Pseudomonas thiazolinophilum* FERM—BP 322 ou *Pseudomonas desmolytica* FERM—BP 323.